# EUROPÄISCHE PATENTANMELDUNG

(11) **EP 2 594 256 A2**
(43) Veröffentlichungstag der Anmeldung: **22.05.2013**
(21) Anmeldenummer: 12181839.7
(22) Anmeldetag: 27.08.2012
(51) Int. Cl.: A61K 8/898, A61Q 5/00, A61Q 5/08, A61Q 5/10

(54) **Vorbehandlungsmittel für keratinische Fasern**

(30) Priorität: 02.09.2011 DE 102011082048
(71) Anmelder: Henkel AG&Co. KGAA, 40589 Düsseldorf (DE)
(72) Erfinder: Schulze zur Wiesche, Erik, 20144 Hamburg (DE); Brockmann, Claudia, 40627 Düsseldorf (DE)

(57) **Zusammenfassung**

Vorbehandlungsmittel für keratinische Fasern, die bezogen auf ihr Gewicht 0,001 bis 1,75 Gew.-% mindestens eines aminofunktionellen Silikons der Formel (I) 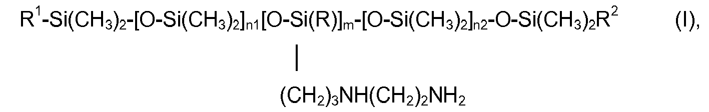 worin
R¹ und R²
unabhängig voneinander ausgewählt sind aus -OH, -O-CH₃, -O-CH₂-CH₃, -CH₃, -CH₂-CH₃, - CH₂-CH₂-CH₃, -CH(CH₃)₂, -C(CH₃)₃
R
ausgewählt ist aus -OH, -O-CH₃, -O-CH₂-CH₃, -CH₃, -CH₂-CH₃, - CH₂-CH₂-CH₃, -CH(CH₃)₂, -C(CH₃)₃
m, n1 und n2
Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt,

sowie mindestens 80 Gew.-% Wasser enthalten, bewirken deutlich verbessertem Haarschutz, ohne dass die Ergebnisse der oxidativen Behandlung beeinträchtigt werden.

## Beschreibung

Die vorliegende Erfindung betrifft kosmetische Zusammensetzungen, die keratinische Fasern vor oxidativen Einflüssen schützen.

Beim Färben und beim Blondieren von Haaren tritt das Problem auf, dass es aufgrund der aggressiven Agentien zu Irritationen auf der Kopfhaut und Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind sogenannte Vorbehandlungsmittel auf dem Markt, die das Haar vor dem aggressiven Einfluss schützen sollen. Diese beschweren das Haar aber oft oder beeinträchtigen den Erfolg der im Anschluss stattfindenden Aufhellung bzw. Färbung des Haares.

Der vorliegenden Erfindung lag die Aufgabe zugrunde, Mittel bereitzustellen, die die genannten Nachteile überwinden, ohne den Erfolg einer nachfolgenden oxidativen Behandlung zu konterkarieren. Dabei sollten insbesondere Mittel bereitgestellt werden, die das Haar nicht beschweren und auch bei einer nicht unmittelbar vor der oxidativen Behandlung stattfindenden Vorbehandlung die gewünschte Wirkung erzielen, wodurch die Zeitspanne zwischen Vorbehandlung und Färben bzw. Blondieren verlängert werden kann.

Der Einsatz von aminierten Silikonen in der Haarpflege ist Stand der Technik. Diese werden in Shampoos und insbesondere in Conditionern breit eingesetzt, um dort Pflegeffekte zu entfalten. So offenbart die EP 1 771 144 B1 haarkonditionierende Mittel mit aminofunktionellen Siliconen. Die dort beschriebenen Mittel sind Nachbehandlungsmittel.

Auch die europäischen Patente EP 1 312 334 B1 (Aminosilicon und Verdicker) sowie EP 1 312 335 B1 (Aminosilicon und Conditioner) offenbaren Haarnachbehandlungsmittel. Im erstgenannten Dokument werden auch extrem wasserreiche Rezepturen offenbart.

Es wurde nun gefunden, dass eine Vorbehandlung der keratinischen Fasern mit gering konzentrierten aminierten Silikonen innerhalb eines bestimmten Zeitraums vor einer oxidativen Behandlung zu deutlich verbessertem Haarschutz führt, ohne dass die Ergebnisse der oxidativen Behandlung beeinträchtigt werden.

Gegenstand der vorliegenden Erfindung sind in einer ersten Ausführungsform Vorbehandlungsmittel für keratinische Fasern, enthaltend bezogen auf ihr Gewicht
a) 0,001 bis 1,75 Gew.-% mindestens eines aminofunktionellen Silikons der Formel 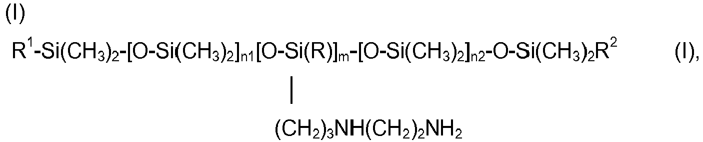 worin
   - R¹ und R²: unabhängig voneinander ausgewählt sind aus -OH, -O-CH₃, -O-CH₂-CH₃, -CH₃, -CH₂-CH₃, - CH₂-CH₂-CH₃, -CH(CH₃)₂, -C(CH₃)₃
   - R: ausgewählt ist aus -OH, -O-CH₃, -O-CH₂-CH₃, -CH₃, -CH₂-CH₃, - CH₂-CH₂-CH₃, -CH(CH₃)₂, -C(CH₃)₃
   - m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt,
b) mindestens 80 Gew.-% Wasser.

Die erfindungsgemäßen Vorbehandlungsmittel sind wasserbasiert und enthalten mindestens 80 Gew.-% Wasser. Bevorzugte erfindungsgemäße Mittel enthalten noch höhere Wassermengen, bis hin zu 99 Gew.-%. Besonders bevorzugte erfindungsgemäße Mittel sind dadurch gekennzeichnet, dass sie, bezogen auf das Gewicht des Mittels, 85 bis 99,9 Gew.%, vorzugsweise 87,5 bis 99,5 Gew.%, besonders bevorzugt 90 bis 99,25 Gew.% und insbesondere 92,5 bis 98 Gew.% Wasser enthalten.

Als Aktivstoff enthalten die erfindungsgemäßen Mittel 0,001 bis 1,75 Gew.-% mindestens eines aminofunktionellen Silikons der Formel (I). Auch das aminofunktionelle Silikon wird vorzugsweise innerhalb engerer Mengenbereiche eingesetzt. Hier sind bevorzugte erfindungsgemäße Mittel dadurch gekennzeichnet, dass sie, bezogen auf das Gewicht des Mittels, 0,05 bis 1,5 Gew.%, vorzugsweise 0,1 bis 1 Gew.%, besonders bevorzugt 0,15 bis 0,75 Gew.% und insbesondere 0,2 bis 0,5 Gew.% aminofunktionelle(s) Silikon(e) enthalten.

In Formel (I) steht n2 vorzugsweise für die Zahl Null, so dass erfindungsgemäß bevorzugte Mittel aminofunktionelle(s) Silikon(e) der Formel (la) anhalten 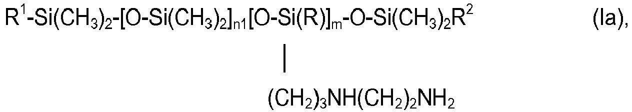 in der die Reste und Indices wie vorstehend definiert sind. R¹ und R² sind unabhängig voneinander ausgewählt sind aus -OH, -O-CH₃, -O-CH₂-CH₃, -CH₃, -CH₂-CH₃, - CH₂-CH₂-CH₃, -CH(CH₃)₂, -C(CH₃)₃. Hierbei sind die Verbindungen der Formel (I) bevorzugt, bei denen R¹ = R² gilt.

R¹ und R² sind vorzugsweise unabhängig voneinander ausgewählt aus -OH und -O-CH₃, so dass erfindungsgemäß bevorzugte Mittel aminofunktionelle(s) Silikon(e) der Formeln (Ib) und/oder (Ic) und/oder (Id) und/oder (Ie) enthalten:

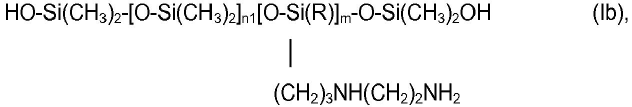

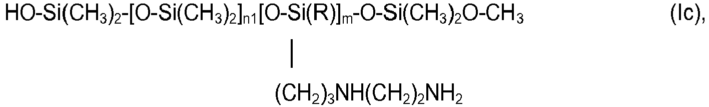

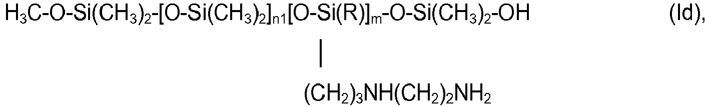

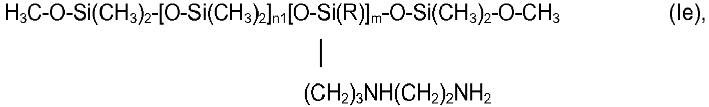

Der Index m steht bevorzugt für eine Zahl zwischen 1 und 1000, vorzugsweise zwischen 50 und 150, der Index n1 steht bevorzugt für eine Zahl zwischen 0 und 999, vorzugsweise zwischen 20 und 700.

Erfindungsgemäß besonders bevorzugte Mittel enthalten demnach aminofunktionelle(s) Silikon(e) der Formeln (Ib) und/oder (Ic) und/oder (Id) und/oder (Ie):

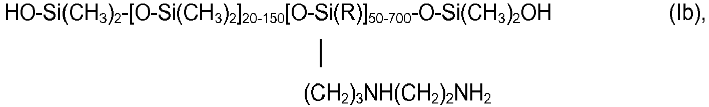

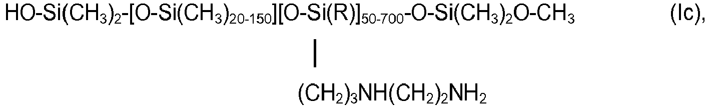

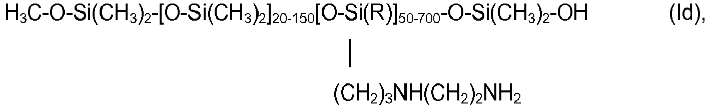

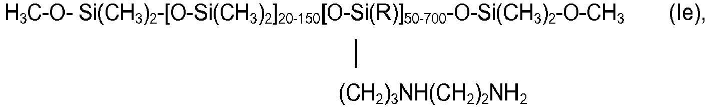

R ist in bevorzugten erfindungsgemäßen Mitteln ausgewählt aus -OH, -O-CH₃, -CH₃. Erfindungsgemäß äußerst bevorzugte Mittel enthalten demnach aminofunktionelle(s) Silikon(e) der Formeln (If) und/oder (Ig) und/oder (Ih) und/oder (Ii) und/oder (Ij) und/oder (Ik) und/oder (I-I) und/oder (Im) und/oder (In) und/oder (Io) und/oder (Ip) und/oder (Iq):

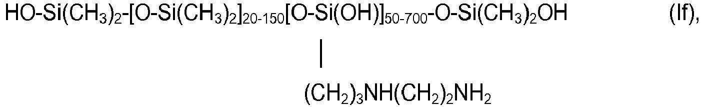

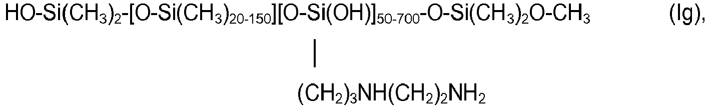

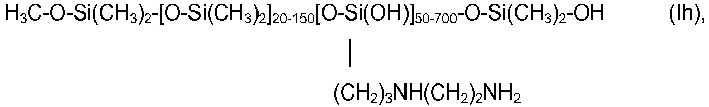

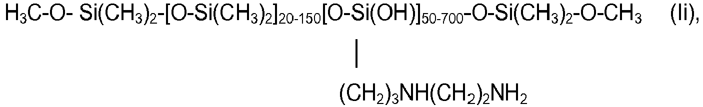

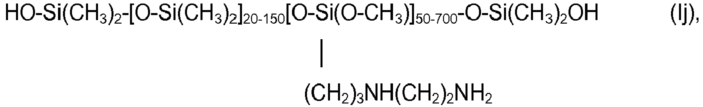

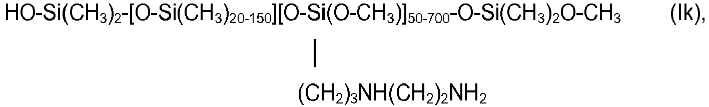

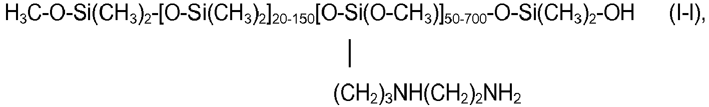

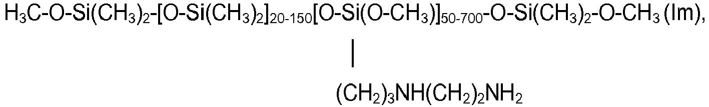

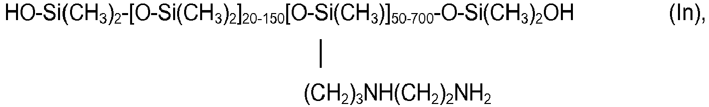

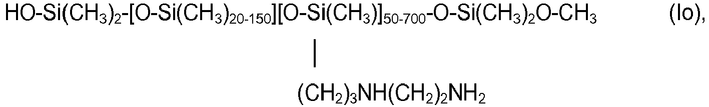

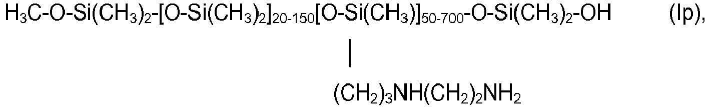

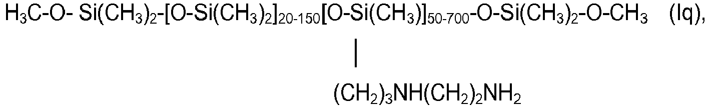

Zusammengefaßt sind erfindungsgemäß bevorzugte Mittel dadurch gekennzeichnet, dass sie aminofunktionelle(s) Silikon(e) der Formel (I) enthalten, in der
- R¹ und R²: unabhängig voneinander ausgewählt sind aus -OH und -O-CH₃
- R: ausgewählt ist aus -OH, -O-CH₃, -CH₃
- m: für eine Zahl zwischen 1 und 1000, vorzugsweise zwischen 50 und 150 steht,
- n1: für eine Zahl zwischen 0 und 999, vorzugsweise zwischen 20 und 700 steht,
- n2: gleich null ist.

Die erfindungsgemäßen Mittel enthalten das Silikon und Wasser, wobei die Einarbeitung des Silikons in die wässrige Grundlage durch Tenside bzw. Emulgatoren erleichtert werden kann. Als besonders vorteilhaft haben sich hierbei Alkoholethoxylate herausgestellt, vorzugsweise ethoxylierte C₃-₂₀-Alkohole mit 1 bis 12 EO. Besonders bevorzugte erfindungsgemäße Mittel sind dadurch gekennzeichnet, dass sie, bezogen auf das Gewicht des Mittels, 0,05 bis 1,5 Gew.%, vorzugsweise 0,1 bis 1 Gew.%, besonders bevorzugt 0,15 bis 0,75 Gew.% und insbesondere 0,2 bis 0,5 Gew.% Alkoholethoxylat(e) der Formel (II) enthalten

H₃C-(CH₂)ₚ-O-(CH₂CH₂-O)_{q}-H (II),

in der
- p: für eine Zahl zwischen 3 und 20, vorzugsweise zwischen 11 und 15 und insbesondere für 12 steht,
- q: für 1, 2, 3, 4, 5, 6, 7, 8, 9, 10, 11, 12, insbesondere für 5 oder 10 steht.

Ganz besonders bevorzugte erfindungsgemäße Mittel enthalten, bezogen auf das Gewicht des Mittels, 0,05 bis 1,5 Gew.%, vorzugsweise 0,1 bis 1 Gew.%, besonders bevorzugt 0,15 bis 0,75 Gew.% und insbesondere 0,2 bis 0,5 Gew.% H₃C-(CH₂)₁₂-O-(CH₂CH₂-O)₅-H oder H₃C-(CH₂)₁₂-O-(CH₂CH₂-O)₁₀-H oder eine Mischung dieser beiden Verbindungen.

Weitere Tenside und Emulgatoren sind in den erfindungsgemäßen Mitteln vorzugsweise nicht oder nur in geringen Mengen enthalten. Erfindungsgemäß bevorzugte Mittel werden tensidarm formuliert, wobei bevorzugte Mittel ≤ 5 Gew.-% Tensid(e), vorzugsweise ≤ 2,5 Gew.-% Tensid(e), weiter bevorzugt ≤ 1 Gew.-% Tensid(e) und insbesondere ≤ 0,5 Gew.-% Tensid(e) enthalten.

In diese Tensidmenge sind die Verbindungen der Formel (II) mit einberechnet.

Vorzugsweise werden die erfindungsgemäßen Mittel niedrigviskos formuliert. Es hat sich darüber hinaus gezeigt, dass verdickende Polymere die erfindungsgemäße Wirkung abschwächen können, so dass bevorzugte erfindungsgemäße Mittel dadurch gekennzeichnet sind, dass sie ≤ 2,5 Gew.-%, vorzugsweise ≤ 1 Gew.-%, weiter bevorzugt ≤ 0,5w.-% und insbesondere ≤ 0,01 Gew.-% verdickende(s) Polymer(e) enthalten.

Mit besonderem Vorzug enthalten die erfindungsgemäßen Mittel unabhängig von der speziellen Wirkung des/der Polymer(e) sie ≤ 2,5 Gew.-%, vorzugsweise ≤ 1 Gew.-%, weiter bevorzugt ≤ 0,5w.-% und insbesondere ≤ 0,01 Gew.-% synthetische(s) Polymer(e)

Die erfindungsgemäßen Vorbehandlungsmittel können weitere Inhaltsstoffe enthalten. Bevorzugt ist hierbei der Einsatz von mehrwertigen Alkoholen, die feuchtigkeitsspendende Eigenschaften aufweisen. Hier sind erfindungsgemäße Mittel bevorzugt, die - bezogen auf ihr Gewicht - 0,05 bis 15 Gew.-%, vorzugsweise 0,1 bis 10 Gew.-%, besonders bevorzugt 0,15 bis 5 Gew.-% und insbesondere 0,15 bis 1 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Glycerin bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Glycerin, so dass Mittel, die außer Glycerin keine anderen mehrwertigen Alkohole enthalten, besonders bevorzugt sind.

Im Hinblick auf die Vorbehandlung vor einer oxidativen Behandlung ist der Einsatz bestimmter Pflegestoffe in den erfindungsgemäßen Mitteln bevorzugt.

Erfindungsgemäß bevorzugte Vorbehandlungsmittel sind dadurch gekennzeichnet, dass sie zusätzlich Pflegestoff(e) - bezogen auf ihr Gewicht - in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe
i. L-Carnitin und/oder seiner Salze;
ii. Taurin und/oder seiner Salze;
iii. Niacinamid;
iv. Ubichinon
v. Ectoin;

L-Carnitin (IUPAC-Name(R)-(3-Carboxy-2-hydroxypropyl)- N,N,N-trimethylammoniumhydroxid), ist eine natürlich vorkommende, vitaminähnliche Substanz. Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich. Erfindungsgemäße bevorzugte Haarbehandlungsmittel sind dadurch gekennzeichnet, dass sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Ein weiterer, bevorzugter einsetzbarer Pflegestoff ist das Taurin. Erfindungsgemäß bevorzugte Vorbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflegestoffen in den erfindungsgemäßen Mitteln sind Vitamine, Provitamine oder Vitaminvorstufen. Diese werden nachfolgend beschrieben: Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt (siehe weiter unten). Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Haarbehandlungsmittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-N-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, dass bestimmte Chinone eine besondere Eignung als Pflegestoff besitzen. Als weiteren Pflegestoff können die erfindungsgemäßen Mittel daher 0,0001 bis 5 Gew.-% mindestens eines Biochinons der Formel (Ubi) 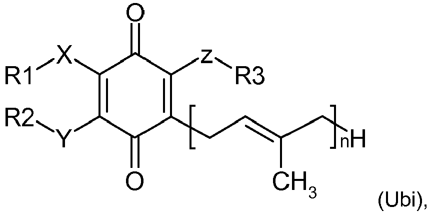 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- R⁴: steht für -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7, 8, 9, 10.

Besonders bevorzugte erfindungsgemäße Vorbehandlungsmittel sind dadurch gekennzeichnet, dass sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 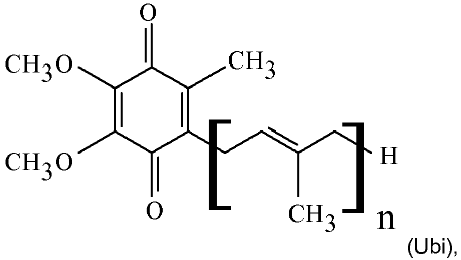 in der n für die Werte = 6, 7, 8, 9 oder 10, besonders bevorzugt für 10 (Coenzym Q10) steht. Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, dass sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten 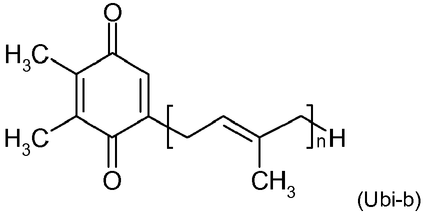 in der n für Werte von 1 bis 20, vorzugsweise von 2 bis 15 und insbesondere für 5, 6, 7, 8, 9, 10 steht, wobei besonders bevorzugt Mittel Plastochinon PQ-9 enthalten.

Als weiteren Pflegestoff können die erfindungsgemäßen Mittel Ectoin enthalten. Erfindungsgemäß bevorzugte Vorbehandlungsmittel sind dadurch gekennzeichnet, dass sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Zur Verbesserung der Elastizität und Festigung der inneren Struktur der mit erfindungsgemäßen Mitteln vorbehandelter Haare können die erfindungsgemäßen Mittel Purin und/oder Purinderivate als Pflegestoff enthalten. Insbesondere die Kombination von Purin und/oder Purinderivaten mit Ubichinonen und/oder Plastochinonen als Pflegestoff führt dazu, dass die nachfolgende oxidative Behandlung schadlos überstanden wird.

Bevorzugte erfindungsgemäße Mittel enthalten Purin und/oder Purinderivate in engeren Mengenbereichen. Hier sind erfindungsgemäß bevorzugte Mittel dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte Vorbehandlungsmittel sind dadurch gekennzeichnet, dass sie als Pflegestoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 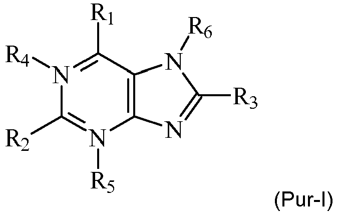 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflegestoff können die erfindungsgemäßen Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflegestoff wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Vorbehandlungsmittel sind dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-O-rutinosid (Rutin), enthalten.

Bevorzugt ist auch der Einsatz von Bisabolol und/oder Bisabololoxiden als Pflegestoff in den erfindungsgemäßen Mitteln. Hier sind erfindungsgemäße Vorbehandlungsmittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflegestoff. Creatin (3-Methylguanidinoessigsäure) ist eine organische Säure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Vorbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% N-Methyl-guanidino-essigsäure (Creatin).

Zusätzlich zu den Pflegestoffen können die erfindungsgemäßen Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, *S*-Methyl-L-methionin, *S*-Allyl-L-cystein-sulfoxid (L-Alliin), *L-trans-4-*Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Haarbehandlungsmittel dadurch gekennzeichnet, dass sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, dass sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose und/oder Disacchariden, insbesondere Saccharose und/oder Maltose und/oder Lactose und/oder Trehalose und/oder Cellobiose und/oder Gentiobiose und/oder Isomaltose.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Wie bereits erwähnt, enthalten bevorzugte erfindungsgemäße Mittel (eine) Aminosäure(n). Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-trans-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Mittel dadurch gekennzeichnet, dass sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat und 0,1 bis 0,25 Gew.-% Glycin,
- 0,005 bis 0,015 Gew.-% Coffein und 0,75 bis 1,5 Gew.-% Saccharose und 0,1 bis 0,25 Gew.-% Glycin,
- 0,005 bis 0,015 Gew.-% Coffein und 0,75 bis 1,5 Gew.-% Fructose und 0,1 bis 0,25 Gew.-% Glycin,
- 0,005 bis 0,015 Gew.-% Coffein und 0,75 bis 1,5 Gew.-% Glucosemonohydrat und 0,1 bis 0,25 Gew.-% Alanin,
- 0,005 bis 0,015 Gew.-% Coffein und 0,75 bis 1,5 Gew.-% Saccharose und 0,1 bis 0,25 Gew.-% Alanin,
- 0,005 bis 0,015 Gew.-% Coffein und 0,75 bis 1,5 Gew.-% Fructose und 0,1 bis 0,25 Gew.-% Alanin,
- 0,005 bis 0,015 Gew.-% Coffein und 0,75 bis 1,5 Gew.-% Glucosemonohydrat und 0,1 bis 0,25 Gew.-% Valin,
- 0,005 bis 0,015 Gew.-% Coffein und 0,75 bis 1,5 Gew.-% Saccharose und 0,1 bis 0,25 Gew.-% Valin,
- 0,005 bis 0,015 Gew.-% Coffein und 0,75 bis 1,5 Gew.-% Fructose und 0,1 bis 0,25 Gew.-% Valin.

Ein weiterer Gegenstand der vorliegenden Erfindung ist ein Verfahren zur oxidativen Haarbehandlung, bei dem
a) ein Vorbehandlungsmittel, welches bezogen auf sein Gewicht 0,001 bis 1,75 Gew.-% mindestens eines aminofunktionellen Silikons der Formel (I) 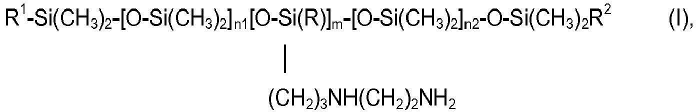 enthält, worin
   - R¹ und R²: unabhängig voneinander ausgewählt sind aus -OH, -O-CH₃, -O-CH₂-CH₃, -CH₃, -CH₂-CH₃, - CH₂-CH₂-CH₃, -CH(CH₃)₂, -C(CH₃)₃
   - R: ausgewählt ist aus -OH, -O-CH₃, -O-CH₂-CH₃, -CH₃, -CH₂-CH₃, -CH₂-CH₂-CH₃, -CH(CH₃)₂, -C(CH₃)₃
   - m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt,
   auf die keratinischen Fasern aufgetragen wird,
b) die keratinischen Fasern innerhalb eines Zeitraumes von einer Sekunde bis 24 Stunden nach Schritt a) einer oxidativen Haarbehandlung unterworfen werden.

Das erfindungsgemäße Verfahren umfaßt das Aufbringen eines Vorbehandlungsmittels auf keratinische Fasern und eine sich daran anschließende oxidative Behandlung. Ein großer Vorteil der in Schritt a) eingesetzten Mittel ist es, dass sie nicht nur dann wirksam sind, wenn sie unmittelbar vor der oxidativen Behandlung angewendet werden, sondern bis zu 24 Stunden vorher angewendet werden können, ohne dass durch äußere Einflüsse eine Abschwächung der Wirkung zu befürchten wäre. Auf diese Weise ist es möglich, beispielsweise morgens nach der Haarwäsche Schritt a) des erfindungsgemäßen Verfahrens durchzuführen und die oxidative Behandlung erst abends.

In besonders bevorzugten erfindungsgemäßen Verfahren ist das in Schritt a eingesetzte Vorbehandlungsmittel ein erfindungsgemäßes Mittel.

Eine oxidative Haarbehandlung kann der Farb- und/oder Formveränderung der keratinischen Fasern dienen. Im Falle der Formveränderung (Dauerwelle, Glätten) wird das Haar vor der oxidativen Behandlung (Fixierung) mit einem weiteren Mittel behandelt, um die Faserstruktur formbar zu machen. In erfindungsgemäß bevorzugten Verfahren dient die oxidative Behandlung der Farbveränderung, stellt also eine Aufhellung ("Blondierung") oder ein Färben dar.

Erfindungsgemäß bevorzugte Verfahren sind dadurch gekennzeichnet, dass Schritt b) das Aufbringen eines Mittels zum Blondieren menschlicher Haare umfaßt, welches mindestens eine Peroxoverbindung enthält, die ausgewählt ist aus Wasserstoffperoxid und dessen Anlagerungsverbindungen an feste Träger, Ammonium- und Alkalimetall-persulfaten und -peroxidisulfaten.

In dem in Schritt b) des erfindungsgemäßen Verfahrens eingesetzten Blondiermittel ist Wasserstoffperoxid enthalten. Bevorzugt wird Wasserstoffperoxid selbst als wässrige Lösung verwendet. Das Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon H₂O₂ (n ist eine positive ganze Zahl größer 0), Harnstoffperoxid und Melaminperoxid, eingesetzt werden.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet. Erfindungsgemäß bevorzugte Verfahren sind dadurch gekennzeichnet, dass die in Schritt b) eingesetzten Mittel - bezogen auf ihr Gewicht - 0,5 bis 12 Gew.-%, vorzugsweise 2 bis 10 Gew.-%, besonders bevorzugt 3 bis 6 Gew.-% Wasserstoffperoxid (berechnet als 100%iges H₂O₂) enthalten.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des die in Schritt b) eingesetzten Mittels zwischen 7 und 11, insbesondere zwischen 8 und 10,5, liegt.

Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe Ammoniak, basischen Aminosäuren, Alkalihydroxiden, Alkanolaminen, Alkalimetallmetasilikaten, Alkaliphosphaten und Alkalihydrogenphosphaten ausgewählt werden. Als Alkalimetallionen dienen bevorzugt Lithium, Natrium, Kalium, insbesondere Natrium oder Kalium.

Die als Alkalisierungsmittel einsetzbaren basischen Aminosäuren werden bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide werden bevorzugt ausgewählt aus der Gruppe Natriumhydroxid und Kaliumhydroxid.

Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Für die starke Aufhellung sehr dunklen Haares ist der Einsatz von Wasserstoffperoxid oder dessen Anlagerungsprodukten an organische bzw. anorganische Verbindungen oftmals nicht ausreichend.

In diesen Fällen wird in der Regel eine Kombination aus Wasserstoffperoxid und Persulfaten eingesetzt.

Die Persulfatsalze können in einer Menge von 0,1 bis 25 g, insbesondere in einer Menge von 1 bis 15 g, bezogen auf 100 g des anwendungsbereiten Mittels, enthalten sein.

Bevorzugte Persulfatsalze sind Ammoniumperoxidisulfat, Kaliumperoxidisulfat, Natriumperoxidisulfat, Ammoniumpersulfat, Kaliumpersulfat und Natriumpersulfat.

Alternativ kann das erfindungsgemäße Verfahren auch bei der Haarfärbung eingesetzt werden. In solchen bevorzugten Ausführungsformen ist das erfindungsgemäße Verfahren dadurch gekennzeichnet, dass Schritt b) das Aufbringen eines Haarfärbemittels umfasst, welches mindestens ein Oxidationsfarbstoffvorprodukt und mindestens ein Oxidationsmittel enthält.

Als ersten zwingenden Inhaltsstoff enthalten die in Schritt b) dieses erfindungsgemäßen Verfahrens eingesetzten Mittel daher mindestens ein Oxidationsfarbstoffvorprodukt. Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige, farbverändernde Verbindungen im erfindungsgemäßen Mittel enthalten sein. In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Ganz besonders bevorzugt ist dabei Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Im Rahmen der vorliegenden Erfindung sind folgende Kombinationen aus Oxidationsfarbstoffvorprodukten vom Entwicklertyp und vom Kupplertyp besonders bevorzugt Mit den als Kombination genannten Oxidationsfarbstoffvorprodukten können jedoch auch noch weitere Farbstoffvorprodukte kombiniert werden:
p-Toluylendiamin / Resorcin;
p-Toluylendiamin / 2-Methylresorcin;
p-Toluylendiamin / 5-Amino-2-methylphenol;
p-Toluylendiamin / 3-Aminophenol;
p-Toluylendiamin / 2-(2,4-Diaminophenoxy)ethanol;
p-Toluylendiamin / 1,3-Bis(2,4-diaminophenoxy)propan;
p-Toluylendiamin / 1-Methoxy-2-amino-4-(2-hydroxyethylamino)benzol;
p-Toluylendiamin / 2-Amino-3-hydroxypyridin;
p-Toluylendiamin / 1-Naphthol;
2-(2-Hydroxyethyl)-p-phenylendiamin / Resorcin;
2-(2-Hydroxyethyl)-p-phenylendiamin / 2-Methylresorcin;
2-(2-Hydroxyethyl)-p-phenylendiamin / 5-Amino-2-methylphenol;
2-(2-Hydroxyethyl)-p-phenylendiamin / 3-Aminophenol;
2-(2-Hydroxyethyl)-p-phenylendiamin / 2-(2,4-Diaminophenoxy)ethanol;
2-(2-Hydroxyethyl)-p-phenylendiamin / 1,3-Bis(2,4-diaminophenoxy)propan;
2-(2-Hydroxyethyl)-p-phenylendiamin / 1-Methoxy-2-amino-4-(2-hydroxyethylamino)benzol;
2-(2-Hydroxyethyl)-p-phenylendiamin / 2-Amino-3-hydroxypyridin;
2-(2-Hydroxyethyl)-p-phenylendiamin / 1-Naphthol;
2-Methoxymethyl-p-phenylendiamin / Resorcin;
2-Methoxymethyl-p-phenylendiamin / 2-Methylresorcin;
2-Methoxymethyl-p-phenylendiamin / 5-Amino-2-methylphenol;
2-Methoxymethyl-p-phenylendiamin / 3-Aminophenol;
2-Methoxymethyl-p-phenylendiamin / 2-(2,4-Diaminophenoxy)ethanol;
2-Methoxymethyl-p-phenylendiamin / 1,3-Bis(2,4-diaminophenoxy)propan;
2-Methoxymethyl-p-phenylendiamin / 1-Methoxy-2-amino-4-(2-hydroxyethylamino)benzol;
2-Methoxymethyl-p-phenylendiamin / 2-Amino-3-hydroxypyridin;
2-Methoxymethyl-p-phenylendiamin / 1-Naphthol;
N-(4-Amino-3-methylphenyl)-N-[3-(1H-imidazol-1-yl)propyl]amin / Resorcin;
N-(4-Amino-3-methylphenyl)-N-[3-(1H-imidazol-1-yl)propyl]amin / 2-Methylresorcin;
N-(4-Amino-3-methylphenyl)-N-[3-(1H-imidazol-1-yl)propyl]amin / 5-Amino-2-methylphenol;
N-(4-Amino-3-methylphenyl)-N-[3-(1H-imidazol-1-yl)propyl]amin / 3-Aminophenol;
N-(4-Amino-3-methylphenyl)-N-[3-(1H-imidazol-1-yl)propyl]amin / 2-(2,4-Diaminophenoxy)ethanol;
N-(4-Amino-3-methylphenyl)-N-[3-(1H-imidazol-1-yl)propyl]amin / 1,3-Bis(2,4-diaminophenoxy)-propan;
N-(4-Amino-3-methylphenyl)-N-[3-(1H-imidazol-1-yl)propyl]amin / 1-Methoxy-2-amino-4-(2-hydroxy-ethylamino)benzol;
N-(4-Amino-3-methylphenyl)-N-[3-(1H-imidazol-1-yl)propyl]amin / 2-Amino-3-hydroxypyridin;
N-(4-Amino-3-methylphenyl)-N-[3-(1H-imidazol-1-yl)propyl]amin/ 1-Naphthol;
4,5-Diamino-1-(2-hydroxyethyl)pyrazol / Resorcin;
4,5-Diamino-1-(2-hydroxyethyl)pyrazol / 2-Methylresorcin;
4,5-Diamino-1-(2-hydroxyethyl)pyrazol / 5-Amino-2-methylphenol;
4,5-Diamino-1-(2-hydroxyethyl)pyrazol / 3-Aminophenol;
4,5-Diamino-1-(2-hydroxyethyl)pyrazol / 2-(2,4-Diaminophenoxy)ethanol;
4,5-Diamino-1-(2-hydroxyethyl)pyrazol / 1,3-Bis(2,4-diaminophenoxy)propan;
4,5-Diamino-1-(2-hydroxyethyl)pyrazol / 1-Methoxy-2-amino-4-(2-hydroxyethylamino)benzol;
4,5-Diamino-1-(2-hydroxyethyl)pyrazol / 2-Amino-3-hydroxypyridin;
4,5-Diamino-1-(2-hydroxyethyl)pyrazol / 1-Naphthol.

Um eine ausgewogene und subtile Nuancenausbildung zu erzielen, ist es erfindungsgemäß vorteilhaft, wenn weitere farbgebende Komponenten in dem Mittel enthalten sind, das in Schritt b) dieser Variante des erfindungsgemäßen Verfahrens eingesetzt wird.

In einer weiteren Ausführungsform können die in Schritt b) dieser Variante des erfindungsgemäßen Verfahrens eingesetzten Mittel zusätzlich mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die Oxidationsmittel, die die Mittel, welche in Schritt b) dieser Variante des erfindungsgemäßen Verfahrens eingesetzt werden, enthalten, sind analog denen, die bei der Blondier-Variante des erfindungsgemäßen Verfahrens beschrieben wurden.

Ein weiterer Gegenstand der vorliegenden Erfindung ist ein Verfahren zum Aufhellen von Keratinfasern, insbesondere menschlichen Haaren, dadurch gekennzeichnet, dass
- ein erfindungsgemäßes Vorbehandlungsmittel auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-60 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird, wobei mindestens eines der Mittel M2, M3 oder M4 mindestens ein Oxidationsmittel mindestens eine Peroxoverbindung, vorzugsweise Wasserstoffperoxid, enthält.

Ein weiterer Gegenstand der vorliegenden Erfindung ist ein Verfahren zum Aufhellen von Keratinfasern, insbesondere menschlichen Haaren, dadurch gekennzeichnet, dass
- ein erfindungsgemäßes Vorbehandlungsmittel auf die Faser aufgebracht wird, dann
- ein Mittel M2 auf der Faser zur Anwendung kommt, das gewünschtenfalls vor der Anwendung aus einem Aufhellmittel M2a und einem Oxidationsmittel M2b gemischt wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird.

Die im erfindungsgemäßen Verfahren eingesetzten Mittel können demnach als Einkomponentenmittel (Mittel M2), oder als Zweikomponenten Mittel (M2a + M2b) formuliert und entsprechend angewendet werden.

Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Aufhellungsverfahren, bei dem das Aufhellmittel M2a und das Oxidationsmittel M2b zunächst getrennt vorliegen, ist dabei bevorzugt.

Für weitere bevorzugte Ausführung gilt mutatis mutandis das zu den erfindungsgemäßen Mitteln Gesagte.

## Patentansprüche

1. Vorbehandlungsmittel für keratinische Fasern, enthaltend bezogen auf sein Gewicht
a) 0,001 bis 1,75 Gew.-% mindestens eines aminofunktionellen Silikons der Formel (I) 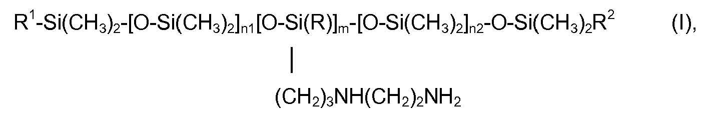 worin
R¹ und R² unabhängig voneinander ausgewählt sind aus -OH, -O-CH₃, -O-CH₂-CH₃, -CH₃, -CH₂-CH₃, - CH₂-CH₂-CH₃, -CH(CH₃)₂, -C(CH₃)₃
R ausgewählt ist aus -OH, -O-CH₃, -O-CH₂-CH₃, -CH₃, -CH₂-CH₃, - CH₂-CH₂-CH₃, -CH(CH₃)₂, -C(CH₃)₃
m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt,
b) mindestens 80 Gew.-% Wasser.

2. Mittel nach Anspruch 1, **dadurch gekennzeichnet, dass** es, bezogen auf das Gewicht des Mittels, 85 bis 99,9 Gew.%, vorzugsweise 87,5 bis 99,5 Gew.%, besonders bevorzugt 90 bis 99,25 Gew.% und insbesondere 92,5 bis 98 Gew.% Wasser enthält.

3. Mittel nach einem der Ansprüche 1 oder 2, **dadurch gekennzeichnet, dass** es, bezogen auf das Gewicht des Mittels, 0,05 bis 1,5 Gew.%, vorzugsweise 0,1 bis 1 Gew.%, besonders bevorzugt 0,15 bis 0,75 Gew.% und insbesondere 0,2 bis 0,5 Gew.% aminofunktionelle(s) Silikon(e) enthält.

4. Mittel nach einem der Ansprüche 1 bis 3, **dadurch gekennzeichnet, dass** es aminofunktionelle(s) Silikon(e) der Formel (I) enthält, in der
R¹ und R² unabhängig voneinander ausgewählt sind aus -OH und -O-CH₃
R ausgewählt ist aus -OH, -O-CH₃, -CH₃
m für eine Zahl zwischen 1 und 1000, vorzugsweise zwischen 50 und 150 steht,
n1 für eine Zahl zwischen 0 und 999, vorzugsweise zwischen 20 und 700 steht,
n2 gleich null ist.

5. Mittel nach einem der Ansprüche 1 bis 4, **dadurch gekennzeichnet, dass** es, bezogen auf das Gewicht des Mittels, 0,05 bis 1,5 Gew.%, vorzugsweise 0,1 bis 1 Gew.%, besonders bevorzugt 0,15 bis 0,75 Gew.% und insbesondere 0,2 bis 0,5 Gew.% Alkoholethoxylat(e) der Formel (II) enthält
H₃C-(CH₂)ₚ-O-(CH₂CH₂-O)_{q}-H (II),
in der
p für eine Zahl zwischen 3 und 20, vorzugsweise zwischen 11 und 15 und insbesondere für 12 steht,
q für 1, 2, 3, 4, 5, 6, 7, 8, 9, 10, 11, 12, insbesondere für 5 oder 10 steht.

6. Mittel nach einem der Ansprüche 1 bis 5, **dadurch gekennzeichnet, dass** es ≤ 5 Gew.-% Tensid(e), vorzugsweise ≤ 2,5 Gew.-% Tensid(e), weiter bevorzugt ≤ 1 Gew.-% Tensid(e) und insbesondere ≤ 0,5 Gew.-% Tensid(e) enthält.

7. Mittel nach einem der Ansprüche 1 bis 6, **dadurch gekennzeichnet, dass** es ≤ 2,5 Gew.-%, vorzugsweise ≤ 1 Gew.-%, weiter bevorzugt ≤ 0,5w.-% und insbesondere ≤ 0,01 Gew.-% verdickende(s) Polymer(e) enthält.

8. Verfahren zur oxidativen Haarbehandlung, **dadurch gekennzeichnet, dass**
a) ein Vorbehandlungsmittel, welches bezogen auf sein Gewicht 0,001 bis 1,75 Gew.-% mindestens eines aminofunktionellen Silikons der Formel (I) 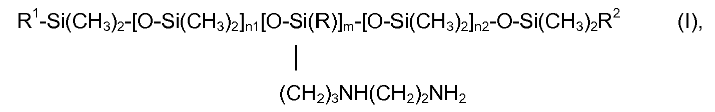 enthält, worin
R¹ und R² unabhängig voneinander ausgewählt sind aus -OH, -O-CH₃, -O-CH₂-CH₃, -CH₃, -CH₂-CH₃, - CH₂-CH₂-CH₃, -CH(CH₃)₂, -C(CH₃)₃
R ausgewählt ist aus -OH, -O-CH₃, -O-CH₂-CH₃, -CH₃, -CH₂-CH₃, -CH₂-CH₂-CH₃, -CH(CH₃)₂, -C(CH₃)₃
m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt,
auf die keratinischen Fasern aufgetragen wird,
b) die keratinischen Fasern innerhalb eines Zeitraumes von einer Sekunde bis 24 Stunden nach Schritt a) einer oxidativen Haarbehandlung unterworfen werden.

9. Verfahren nach Anspruch 8, **dadurch gekennzeichnet, dass** das in Schritt a eingesetzte Vorbehandlungsmittel ein Mittel nach einem der Ansprüche 1 bis 7 ist.

10. Verfahren nach einem der Ansprüche 8 oder 9, **dadurch gekennzeichnet, dass** Schritt b) das Aufbringen eines Mittels zum Blondieren menschlicher Haare umfaßt, welches mindestens eine Peroxoverbindung enthält, die ausgewählt ist aus Wasserstoffperoxid und dessen Anlagerungsverbindungen an feste Träger, Ammonium- und Alkalimetall-persulfaten und -peroxidisulfaten.

11. Verfahren nach einem der Ansprüche 8 oder 9, **dadurch gekennzeichnet, dass** Schritt b) das Aufbringen eines Haarfärbemittels umfaßt, welches mindestens ein Oxidationsfarbstoffvorprodukt und mindestens ein Oxidationsmittel enthält.

12. Verfahren zum Aufhellen von Keratinfasern, insbesondere menschlichen Haaren, **dadurch gekennzeichnet, dass**
- ein Vorbehandlungsmittel nach einem der Ansprüche 1 bis 7 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-60 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird, wobei mindestens eines der Mittel M2, M3 oder M4 mindestens ein Oxidationsmittel mindestens eine Peroxoverbindung, vorzugsweise Wasserstoffperoxid, enthält.
